Europäisches Patentamt

European Patent Office

Office européen des brevets

(19)

(11) Veröffentlichungsnummer: **0 268 700**
**A1**

(12) # EUROPÄISCHE PATENTANMELDUNG

(21) Anmeldenummer: 86116395.4

(22) Anmeldetag: 26.11.86

(51) Int. Cl.⁴: **A61M 5/14**

(43) Veröffentlichungstag der Anmeldung:
**01.06.88 Patentblatt 88/22**

(84) Benannte Vertragsstaaten:
**ES**

(71) Anmelder: **Bischof, Reinhard**
**Lerchenweg 11**
**D-8088 Eching(DE)**

(72) Erfinder: **Bischof, Reinhard**
**Lerchenweg 11**
**D-8088 Eching(DE)**

(74) Vertreter: **Popp, Eugen, Dr. et al**
**MEISSNER, BOLTE & PARTNER**
**Widenmayerstrasse 48 Postfach 86 06 24**
**D-8000 München 86(DE)**

(54) **Mehrwegeventil für eine Anordnung zur Herstellung oder Verabreichung von Infusionslösungen.**

(57) Anordnung zur Verabreichung und/oder Herstellung von Medikament-und/oder Nährlösungen, insbesondere parenteralen Nährlösungen, mit einem Mehrwegeventil (10) dessen Einlaß mit individuell absperrbaren Schlauchverbindungen (12, 14, 16, 18) zum Anschluß an mindestens zwei Vorratsbehälter (20) verbunden und dessen Auslaß (30) an einen Patiente (P) anschließbar ist. Gegebenenfalls weist das Mehrwegeventil (10) einen weiteren sowohl als Ein-als auch Auslaß wirksamen Anschluß (40) auf, an den eine vorzugsweise spritzenartige Saugeinrichtung (42) anschließbar ist.

Zur präzisen bzw. kontrollierten Verabreichung von Infusionen und zur Vermeidung von Lösungs-Mischungen in den zu den Vorratsbehältern führenden Schlauchverbindungen umfaßt der Einlaß des Mehrwege-Ventils (10) für jeden Vorratsbehälter (20) bestimmte Einzel-Einlässe (22, 24, 26, 28), wobei jedem Einzel-Einlaß ein nur in Richtung vom Einlaß zum Auslaß (30) hin öffenbares Rückschlagventil (22', 24', 26', 28') zugeordnet ist.

EP 0 268 700 A1

## Mehrwegeventil für eine Anordnung zur Herstellung oder Verabreichung von Infusionslösungen

Die Erfindung betrifft ein Mehrwegeventil nach dem Oberbegriff des Patentanspruches 1.

Aus der DE-PS 3 024 768 ist eine Anordnung zur Herstellung parenteraler Nährlösungen bekannt, wobei über das dort auch vorgesehene Mehrwegeventil nur wenig gesagt ist. In jedem Fall weist das bekannte Mehrwegeventil nur einen einzigen Einlaß auf, an dem die individuell absperrbaren Schlauchverbindungen zu den Vorratsbehältern anschließbar sind. Zu diesem Zweck sind die Schlauchverbindungen vor dem Einlaß des Mehrwegeventils zu einem Anschlußstück vereinigt.

Bei dieser bekannten Anordnung tritt das Problem auf, daß die Anzahl der anzuschließenden Vorratsbehälter relativ beschränkt ist und die Anordung nicht zur Verabreichung von Infusionen verwendet werden kann, da vor dem einzigen Ventileinlaß die Gefahr einer unkontrollierten Vermischung der verschiedenen Lösungen aus den Vorratsbehältern besteht. Insbesondere dreht es sich hier um die Gefahr der osmotischen Vermischung in den einzelnen Schlauchverbindungen.

Aus dem deutschen Gebrauchsmuster 7 814 016 ist eine Anordnung bekannt, bei der ebenfalls die Gefahr der Vermischung der in einen Schlauchabschnitt eingeleiteten Lösung in den Anschluß-Leitungsabschnitten besteht. Darüber hinaus kommt es bei dieser Anordnung relative leicht zu einer Fehlbedienung der dort vorgesehenen Schiebeklemmen mit der Folge, daß unerwünschte Überdosen einzelner Lösungen erhalten werden.

Aus der US-PS 4 105 029 ist ein Mehrwegeventil bekannt, bei dem Rückschlagventile den Einzel-Einlässen zugeordnet sind. Ein Vermischen der verschiedenen Lösungen wird auch hier nicht sicher verhindert, vielmehr muß das Bedienungspersonal die Beutel mit den Infusionslösungen in exakt definierte Höhen hängen, um dieser Vermischung vorzubeugen.

Ausgehend vom oben genannten Stand der Technik liegt der vorliegenden Erfindung die Aufgabe zugrunde, ein Mehrwegeventil der eingangs genannten Art dahingehend weiterzubilden, daß eine unzulässige Vermischung verschiedener Infusionslösungen, auch durch Fehlbedienung, sicher verhindert wird.

Diese Aufgabe wird durch die im Kennzeichen des Patentanspruches 1 genannten Merkmale gelöst.

Selbstverständlich wird hierbei gleichzeitig auch die hier notwendige Sterilisierbarkeit und die gute Dosierfähigkeit der Anordnung gewährleistet, wie dies bei Geräten der hier behandelten Art immer notwendig ist.

Das Mehrwegeventil kann sowohl zur Herstellung, als auch zur Verabreichung der Infusionslösungen verwendet werden oder aber auch zu einer gleichzeitigen Herstellung und Verabreichung.

Es kann also mit dem vorliegenden Mehrwegeventil auf funktionssichere Weise eine Vielzahl unterschiedlicher Infusionslösungen verabreicht werden, wie z. B. eine parenterale Nährlösung, ein Elektrolyt, Bicarbonat, Gamma-Globolin, Fett, Blut, Lidocain etc.

Vorzugsweise ist einigen Schlauchverbindungen eine Infusionspumpe zugeordnet, um z. B. während einer Operation bei plötzlich auftretenden Komplikationen über diese Pumpe mehr oder weniger Lidocain und/oder Bicarbonat dem Patienten verabreichen zu können.

Eine individuelle zusätzliche Verabreichung von Einzellösungen kann auch durch Ansaugen der einen oder anderen Lösung über den sowohl als Einals auch als Auslaß dienenden Anschluß des Mehrwegeventils mittels einer spritzenartigen Pumpeinrichtung erfolgen, wobei die Verabreichung über den Auslaß des Mehrwegeventils erfolgt. Eine unkontrollierte "Rückmischung" der angesaugten Teilmengen bei der Verabreichung ist durch die, jedem Einzel-Einlaß zugeordneten Rückschlage nicht möglich.

Der sowohl als Ein-als auch Auslaß dienende Anschluß kann auch zur (herkömmlichen) Herstellung parenteraler Nährlösungen verwendet werden, wobei dann der Auslaß des Mehrwegeventils mit einem Mischbeutel verbunden ist.

Die Sterilisierbarkeit, die in dem hier vorliegenden Verwendungsbereich unbedingt gegeben sein muß, wird dadurch verbessert, daß keinerlei mechanisch zu betätigende Drehhähne mehr vorgesehen sind. Somit treten die bisher üblichen Schwierigkeiten bei Gassterilisation oder auch Gamma-Sterilisation nicht mehr auf, die durch die Gleit-und Dichtflächen (Silikonfilm) bisher auftraten.

Vorzugsweise mündet in den Sammelraum des Mehrwegeventils noch ein Injektionsstutzen zum Injizieren von vorgegebenen Mengen eines bestimmten Medikaments.

Ein ganz wesentlicher Vorteil der Anordnung besteht darin, daß eine Vermischung von Einzellösungen auch dann nicht erfolgen kann, wenn versehentlich vergessen wurde, die benötigten Schlauchverbindungen mittels Schlauchklemmen abzusperren. Eine Unterbrechung der Schlauchverbindung ist bei der hier gezeigten Anordnung nur dann erforderlich, wenn die entsprechend zugeordnete Infusionslösung nicht verabreicht oder angesaugt werden soll. Dies ist vor allem bei kritischen Operationen in der hektischen Atmosphäre des Operationssaales von großer Wichtigkeit und war bisher nicht gegeben.

Das Mehrwegeventil läßt sich äußerst kompakt herstellen, wenn man z. B. die Einzeleinlässe auch in Reihe sehr eng hintereinander anordnet. Darüber hinaus ist es natürlich auch möglich, die Einzeleinlässe sternförmig zu verteilen, woebi die Reihenanordnung jedoch eine größere Übersichtlichkeit sicherstellt.

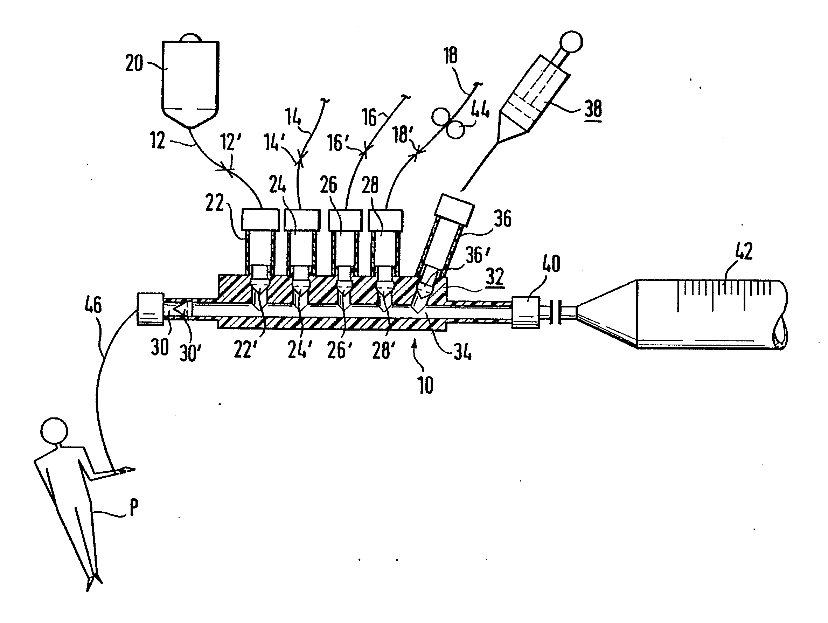

Nachstehend wird eine bevorzugte Ausführungsform des Mehrwege-Ventils anhand der beigefügten Zeichnung näher erläutert. Diese zeigt im schematischen Teilschnitt und schematischer Teilansicht eine Anordnung zur Verabreichung oder Herstellung von Medikament-und/oder Nährlösungen, insbesondere parenteralen Nährlösungen, mit einem Mehrwege-Ventil 10, an das mehrere, hier vier, Vorratsbehälter z.B. für einen Elektrolyt, Bicarbonat, Gamma-Globulin und Lidocain über Schlauchverbindungen 12, 14, 16, 18 angeschlossen sind. In der Zeichnung ist nur ein Vorratsbehälter dargestellt. Dieser ist mit der Bezugsziffer 20 bezeichnet. Die Schlauchverbindungen 12...18 sind jeweils individuell mittels Schlauchklemmen 12', 14', 16' und 18', vorzugsweise Rollenklemmen, absperrbar bzw. abklemmbar. Jeder Schlauchverbindung 12...18 ist ein gesonderter Einlaß 22, 24, 26, 28 zugeordnet, wobei jeder Einzeleinlass ein nur in Richtung vom Einlass zu einem Auslass 30 hin öffenbares Rückschlagventil 22', 24', 26' und 28', nämlich Schnabelventil, umfaßt. Auch der Auslaß weist ein nur in Richtung von den Einzel-Einlässen 22...28 zum Auslaß 30 hin öffenbares Rückschlagventil 30', nämlich Schnabel-, ventil auf. Dadurch ist sichergestellt, daß vom Patienten P kein Infusions-Rückfluß stattfindet.

Die Einzel-Einlässe 22...28 münden jeweils in einen gemeinsamen, innerhalb des Ventilkörpers 32 angeordneten Sammelraum 34 in Form eines durchgehenden Kanals.

In diesen Sammelraum bzw. Kanal 34 mündet ferner ein Injektionsstutzen 36 herkömmlicher Art zum Injizieren einer vorgegebenen Menge eines Arzneimittels mittels einer Spritze 38. An den Sammelraum bzw. Kanal 34 ist ferner der Zum Patienten P führende Auslaß 30 sowie ein als Ein-und Auslaß wirksamer Anschluß 40 angeschlossen. Dieser Anschluß 40 weist kein Rückschlagventil auf. Er ist mittels einer Kappe oder eines Sperrhahns (nicht dargestellt) verschließbar. Bei Bedarf ist an diesen Anschluß eine Saugeinrichtung z.B. in Form einer Saugspritze bzw. Perfusorspritze 42, anschließbar. Mittels dieser Spritze können Teilmengen aus den Vorratsbeuteln 20 angesaugt und dem Patienten vorzugsweise zeitgesteuert verabreicht werden. Zur Herstellung einer parenteralen Nährlösung ist der Auslaß 30 mit einem nicht dargestellten Mischbehälter,z.B. Mischbeutel verbunden. Insofern ist das dargestellte Mehrwege-Ventil universell verwendbar.

Einer oder mehreren Schlauchverbindungen 12, 14, 16, 18 kann bzw. können Infusionspumpen zugeordnet sein. Bei dem dargestellten Ausführungsbeispiel ist der Schlauchverbindung bzw. -leitung 18 eine Infusions-Pumpe 44 zugeordnet. Diese ist in der Zeichnung nur schematisch dargestellt.

Die Einzel-Einlässe 22..28 weisen jeweils sogenannte Luer-Anschlüsse auf zur Ankoppelung der Schlauchverbindungen bzw. -leitungen 12..18. Im übrigen sind die Einzel-Einlässe 22..28 als flexible Anschlußstutzen ausgebildet, wodurch eine enge Aneinanderreihung möglich ist. Bei Bedarf können sie geringfügig voneinander weggebogen werden, um sich nicht gegenseitig beim Anschluß der Schlauchverbindungen 12..18 zu behindern. Grundsätzlich ist es natürlich denkbar, die Einzel-Einlässe 22..28 über den Umfang des Sammelraums bzw. Durchgangskanals 34 verteilt anzuordnen. Es hat sich in der Praxis jedoch gezeigt, daß die Anordnung der Einzel-Einlässe 22..28 in Reihe hintereinander die beste Übersichtlichkeit und Kontrolle der Gesamtanordnung vermittelt.

Von Bedeutung ist noch, daß es sich bei der erfindungsgemäßen Anordnung um ein geschlossenes System handelt. Es besteht demnach auch keine Kotaminationsgefahr. Auch der Auslaß 30 sowie Anschluß 40 weist jeweils eine sogenannte Luer-Kupplung auf.

Konkret sind die Rückschlagventile 22'..30' im Ventilkörper eingesetzt. Auch dem Injektionsstutzen 36 ist innerhalb des Ventilkörpers 32 ein Rückschlagventil 36' zugeordnet, das nur in Richtung vom Injektionsstutzen zum Auslaß 30 hin öffenbar ist. Die vom Auslaß 30 zum Patienten P führende Patientenleitung ist in der anliegenden Zeichnung mit der Bezugsziffer 46 gekennzeichnet.

Mittels der Spritze 42 kann auch während einer Operation eine spezielle Infusionsmischung hergestellt werden, die dann beschleunigt dem Patienten verabreicht wird. Von dieser Maßnahme wird insbesondere dann Gebrauch gemacht, wenn Komplikationen während einer Operation auftreten.

Das Mehrwege-Ventil läßt sich äußerst kompakt herstellen. Die Anzahl der Einzel-Einlässe ist im Grunde genommen unbegrenzt, wobei derzeit maximal bis zu acht Einlässe benötigt werden. Um die Mehrwege-Ventile nicht zu groß bauen zu müssen, können auch zwei oder mehr Mehrwege-Ventile der dargestellten Art parallel zueinander geschaltet werden. Die Auslässe werden dann über ein Y-Schlauchverbindungsstück zusammengeführt, bevor der Anschluß an die Patientenzuleitung 46 erfolgt.

Auch ohne Abklemmung der Schlauchverbindungen 12..18 besteht nicht die Gefahr, daß die eine Infusionslösung in eine andere Schlauchverbindung bzw. -leitung eindringt. Dies wird selbsttätig verhindert durch die Rückschlagventile 22'..28'.

## Ansprüche

1. Mehrwegeventile für eine Anordung zur Herstellung und Verabreichung von Infusionslösungen, dessen Einlaß mit individuell absperrbaren Schlauchverbindungen (12, 14, 18) zum Anschluß an mindestens zwei Vorratsbehälter (20) verbunden und dessen Auslaß (30) an einen Patienten (P) anschließbar ist, und das einen weiteren Anschluß - (40) aufweist, an den eine Saugeinrichtung (42) anschließbar ist, wobei ein Ventilkörper (32) vorgesehen ist, **dadurch gekennzeichnet,** daß das Mehrwege Ventil (10) einen in dem Ventilkörper (32) angeordneteten Sammelraum (34) umfaßt, in den mehrere jeweils für einen Vorratsbehälter (20) bestimmte Einzel-Einlässe (22 ... 28), der Auslaß (30), sowie der zusätzliche, sowohl als Ein-als auch als Auslaß benutzbare Anschluß (40) vorgesehen ist, wobei jeder Einzel-Einlaß (22 ... 28) ein nur zum Auslaß (30) hin öffenbares Rückschlagventil (22', 24', 26', 28') aufweist.

2. Mehrwegeventil nach Anspruch 1, **dadurch gekennzeichnet,** daß der sowohl als Ein-als auch als Auslaß benutzbare Ventilanschluß - (40) bei Bedarf absperrbar ist, insbesondere durch eine Kappe oder einen Sperrhahn.

3. Mehrwegeventil nach einem der vorhergehenden Ansprüche, **dadurch gekennzeichnet,** daß dem Auslaß (30) ebenfalls ein nur in Richtung vom Einlaß (22 ... 28) zum Auslaß (30) bzw. Patient (P) hin öffenbares Rückschlagventil (30'), insbesondere Schnabelventil, zugeordnet ist.

4. Mehrwegeventil nach einem der vorhegehenden Ansprüche, **dadurch gekennzeichnet,** daß die Schlauchverbindungen (12 ... 18) zu den Vorratsbehältern (20) mittels Schlauchklemmen (12', 14', 16', 18') absperrbar sind.

5. Mehrwegeventil nach einem der vorhergehenden Ansprüche, **dadurch gekennzeichnet,** daß das Mehrwegeventil (10) noch einen gesonderten Injektionsstutzen (36) aufweist, dem vorzugsweise ebenfalls ein nur zum Auslaß (30) hin öffenbares Rückschlagventil (36') zugeordnet ist.

6. Mehrwegeventil nach einem der vorhergehenden Ansprüche, **dadurch gekennzeichnet,** daß die Einzel-Ein lässe (22 ... 28) Luer-Kupplungen aufweisen, die jeweils über flexible schlauchartige Verbindungsstutzen mit dem Ventilkörper (32) verbunden sind.

7. Mehrwegeventil nach einem der vorhergehenden Ansprüche, **dadurch gekennzeichnet,** daß jedem Einzel-Einlaß (22 ... 28) mindestens zwei Vorratsbehälter zugeordnet sind, wobei die Schlauchverbindungen zu diesen Vorratsbehältern vor dem jeweiligen Einzel-Einlaß zu einem gemeinsamen Anschlußstück vereinigt sind.

8. Mehrwegeventil nach einem der vorhergehenden Ansprüche, **dadurch gekennzeichnet,** daß einer oder mehreren Schlauchverbindungen (12 ... 18) eine Infusionspumpe (44) zugeordnet ist.

0 268 700

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| E | DE-A-3 520 044 (BAXTER TRAVENOL LABORATORIES INC.) * ganzes Dokument * --- | 1-8 | A 61 M 5/14 |
| Y | DE-A-2 630 050 (PALEY) * Ansprüche 1-4; Seite 7, Zeilen 23-32; Figuren 1, 14 * | 1,5 | |
| A | --- | 6 | |
| Y | FR-A-2 306 711 (BIEDERMANN) * Ansprüche 1-3; Figuren 2, 5 * --- | 1,5 | |
| A | WO-A-8 400 340 (BAXTER TRAVENOL LABORATORIES INC.) * Anspruch 1; Figuren 1, 6 * ----- | 1 | |

RECHERCHIERTE SACHGEBIETE (Int. Cl.4)

A 61 M 5/00

Der vorliegende Recherchenbericht wurde für alle Patentansprüche erstellt.

| Recherchenort | Abschlußdatum der Recherche | Prüfer |
|---|---|---|
| BERLIN | 17-07-1987 | PAPA E.R. |